# EUROPEAN PATENT APPLICATION

(11) **EP 4 711 763 A1**
(43) Date of publication of application: **18.03.2026**
(21) Application number: 25202090.4
(22) Date of filing: 15.09.2025
(51) Int. Cl.: G01N 33/68

(54) **DETECTING XENOTRANSPLANT ORGAN REJECTION**

(30) Priority: 13.09.2024 US 202463694451 P
(71) Applicant: Immucor GTI Diagnostics, Inc., Waukesha, WI 53186 (US)
(72) Inventor: RAY, BRYAN, WAUKESHA 53186 (US); HARIHARAN, JAYASREE, WAUKESHA 53186 (US)
(74) Representative: ZBM Patents - Zea, Barlocci & Markvardsen

(57) **Abstract**

Described herein are kits, compositions, and methods for detecting porcine antigens in biological samples. The kit comprises a support, anti-porcine antibodies coupled to the support, and instructions for preparing lysates from a porcine donor source and contacting the lysates with the anti-porcine antibodies. The kit may be for use in determining the risk of transplant rejection in a human subject receiving or being considered for receiving a transplant from the porcine donor.

## Description

### CROSS-REFERENCE TO RELATED APPLICATION

This application claims the benefit of, and priority to, the earlier filing date of U.S. Provisional Patent Application No. 63/694,451, which was filed September 13, 2024, and which is titled "Detecting Xenotransplant Organ Rejection". U.S. Provisional Patent Application No. 63/694,451 is incorporated herein by reference in its entirety.

### TECHNICAL FIELD

This specification relates detecting xenotransplant organ rejection.

### BACKGROUND

Porcine to human xenotransplantation is a promising approach for addressing the critical shortage of human organs available for transplant. Because of genetic differences between pigs and humans, humans may develop antibodies directed against xenoantigens leading to antibody-mediated rejection - a major cause of graft injury and transplant rejection.

Current methods look at the ability of pig red blood cells or human embryonic kidney cells expressing recombinant swine leukocyte antigens (SLA) to bind antibodies in sera. There is currently no single assay that can simultaneously detect and identify antibodies directed against a multitude of porcine antigens including SLA.

### SUMMARY

An example kit for use in detecting porcine antigens in a biological sample from a human. The kit may comprise a support and anti-porcine antibodies coupled to the support. The kit may comprise instructions for preparing lysates from a porcine donor source and contacting the lysates with the anti-porcine antibodies. The kit may be for use in determining the risk of transplant rejection in a human subject receiving or being considered for receiving a transplant from the porcine donor. In some implementations, the porcine donor source is from peripheral blood, spleen or tissue of the porcine donor.

The support may comprise a well, a bead, or a patterned surface. In some implementations, the kit may further comprise a human antibody having a label attached thereto. The kit may have anti-porcine antibodies that are configured to bind to a porcine antigen present in a lysate from a porcine donor source. The kit may contain lysis buffer for preparing a lysate from a porcine donor source. The kit may be provided with a wash buffer.

Optionally, the support comprises one or more isolated surfaces each surface containing a population of the anti-porcine antibodies, each population of anti-porcine antibody distinct from each other.

In some implementations, anti-porcine antibodies are selected from anti-porcine antibodies directed against one or more of Swine Leukocyte Antigen (SLA) class II DR, SLA Class II DQ, SLA Class I, an SLA subclass selected from SLA1, SLA2, SLA3, SLA6, SLA7, or SLA8, or a non-SLA (optionally, MIC2), or selected from BL2H5, BL4H2, 2F4, 1F12, 2E9/13, JM1E3, or 12E7.

In some implementations, a first population of anti-porcine antibodies is directed against SLA class II DR, a second population of porcine antibodies is directed against SLA Class II DQ, and a third population of porcine antibodies is directed against SLA Class I or an SLA subclass selected from SLA1, SLA2, SLA3, SLA6, SLA7, or SLA8. Optionally, the first population comprises an antibody selected from BL2H5 or BL4H2; optionally, the second population comprises an antibody selected from 2F4, 1F12 or 2E9/13; and optionally the third population comprises an antibody selected from JM1E3. In some implementations, a fourth population of anti-porcine antibodies is directed against a non-SLA. Optionally, the non-SLA is the antigen MIC2 and the fourth population is the antibody 12E7.

An example composition includes one or more populations of anti-porcine antibodies coupled to one or more surfaces. For example, the composition may include a first population of anti-porcine antibodies coupled to a first surface, a second population of anti-porcine antibodies coupled to a second surface, and a third population of anti-porcine antibodies coupled to a third surface. Optionally, the composition further includes a fourth population of anti-porcine antibodies coupled to a fourth surface. Optionally the first population of anti-porcine antibodies is directed against SLA class II DR, the second population or anti-porcine antibodies is directed against SLA Class II DQ, and the third population of anti-porcine antibodies is directed against SLA Class I or an SLA subclass selected from SLA1, SLA2, SLA3, SLA6, SLA7, or SLA8, the fourth population of porcine antibodies is directed against MHC proteins. The composition may further include first porcine antigens from a single porcine donor bound to the first population of anti-porcine antibodies, second porcine antigens from the single porcine donor bound to the second population of anti-porcine antibodies, and third porcine antigens from the single porcine donor bound to the third population of anti-porcine antibodies. Optionally, the composition includes fourth porcine antigens from a single porcine donor bound to the fourth population of anti-porcine antibodies. In some implementations, the composition may be provided with the other elements of the kit as described herein. In some implementations, the composition may be provided with an anti-human antibody having a label attached thereto. In some implementations, the composition may be provided with anti-porcine antibodies that are configured to bind to a porcine antigen present in a lysate from a porcine donor source.

An example method of detecting one or more anti-porcine antibodies in a biological sample from a human. The method includes: (a) contacting porcine lysate containing porcine antigens with the composition described herein; (b) removing any unbound porcine antigen; (c) contacting a human biological sample containing human antibodies with the composition of step (b); (d) removing any unbound human antibodies; (e) contacting the composition of step d with a secondary antibody such as antibodies directed against either human IgG or human IgM, the secondary antibody including a label; (f) detecting the label: and (g) determining the relative or absolute amount of anti-porcine antibodies in the sample based on the level of the detected label. The label may be an enzyme or a dye. Optionally, the biological sample is sera, plasma, lymph fluid, or biopsy aspirates. Optionally, the porcine lysate is sourced from peripheral blood, spleen or tissue of a single porcine donor.

At least part of the systems and techniques, including methods, described in this specification may be implemented or controlled by executing, on one or more processing devices, instructions that are stored on one or more non-transitory machine-readable storage media. Examples of non-transitory machine-readable storage media include read-only memory, an optical disk drive, memory disk drive, and random-access memory. At least part of the systems and techniques described in this specification may be implemented or controlled using a computing system comprised of one or more processing devices and memory storing instructions that are executable by the one or more processing devices to perform various control operations. The compositions, devices, systems, and techniques described in this specification may be configured, for example, through design, construction, formulation, arrangement, placement, programming, operation, activation, deactivation, and/or control.

Any two or more of the features described in this specification, including in this summary section, may be combined to form implementations not specifically described in this specification.

The details of one or more implementations are set forth in the accompanying drawings and the description below. Other features and advantages will be apparent from the description and drawings, and from the claims.

### DESCRIPTION OF THE DRAWINGS

FIG. 1 illustrates compositions for detecting donor specific antigens, according to some implementations.
FIG. 2 shows populations of anti-porcine antibodies, according to some implementations.
FIG. 3 is a flowchart showing operations or steps included in a process for detecting antibodies, according to some implementations.

Like reference numerals in different figures indicate like elements.

### DETAILED DESCRIPTION

Described herein are kits, compositions, and methods for detecting porcine antigens in biological samples. These kits, compositions, and methods are for donor specific assays where a human xenograft recipient of a porcine donor organ is monitored for compatibility with the transplanted organ, or a porcine donor is screened for compatibility to a human recipient or potential recipient. This contrasts with methods that are not specific to a single porcine donor and where the human recipient is screened against a general population of the porcine donor. In such non donor specific assays, the risk of organ rejection is increased because a specific donor antigen may not be present on the panel.

The kits, compositions, and methods may be used to determine whether a human recipient or potential recipient has antibodies that react with the porcine antigens. While porcine leukocyte antigens are different from human leukocyte antigens, they share some homology, so it is possible for the human recipient or potential recipient to have antibodies that react with porcine antigens. If a potential human recipient has antibody against a potential porcine donor, the transplant may preferably not be performed using that porcine donor. In some cases, for example, where a potential recipient does not have better alternatives, and if there are several porcine donors, then the porcine donor with the least amount of reactivity may be selected. For example, screening may be performed among several porcine lines, to identify which line or lines has the least amount of reactivity with the potential human recipient. Additionally, where the potential human recipient has antibody against the porcine donor, the potential human recipient may be treated to reduce or eliminate the antibodies prior to transplant.

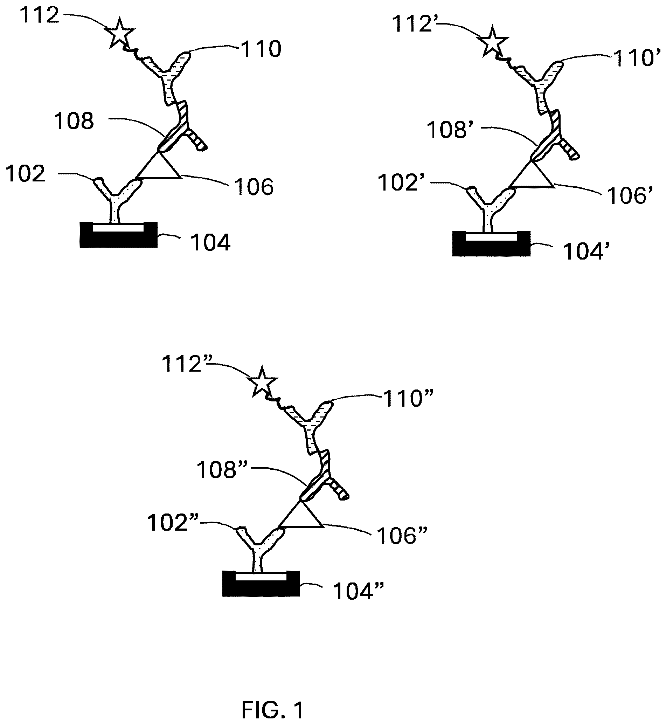

FIG. 1 illustrates compositions for detecting donor specific antigens. The compositions include a first porcine antibodies 102 coupled to a first support 104. First porcine antigens 106 are coupled to the first porcine antibodies 102 by antigen-antibody interactions. The first porcine antigens 106 can form complexes with first human antibodies 108 having an affinity for the first porcine antigens 106. The first human antibodies 108 are detected with a first secondary antibody 110 which includes a first label 112 attached thereto.

In some implementation additional supports such as a second support 104' and a third support 104" are used, where the second and third supports optionally can have the same composition or form. Second porcine antibodies 102' can be coupled to the support 104', and third porcine antibodies 102" can be coupled to the support 104", wherein antibodies 102, 102' and 102" are different from each other. Second porcine antigens 106' can be coupled to the porcine antibodies 102' by antigen-antibody interactions and third porcine antigens 106" can be coupled to the porcine antibodies 102", where one or more of the porcine antigens 106, 106', and 106" can be different from each other. Second porcine antigens 106' can form complexes with a second human antibodies 108', and third porcine antigens 106" can form complexes with third human antibodies 108". The human antibodies 108, 108', and 108" can be different from each other. The second human antibodies 108' are detected with a second secondary antibody 110' which includes a second label 112' attached thereto. The third human antibodies 108" are detected with a third secondary antibody 110" which includes a third label 112" attached thereto. The secondary antibodies 110, 110' and 110" and labels 112, 112' and 112" are the same.

In some other implementations fourth, fifth, sixth or more supports, porcine antibodies, porcine antigens, anti-porcine human antibodies, secondary antibodies, and labels are also used. The fourth, fifth, sixth or more supports, porcine antibodies, porcine antigens, anti-porcine human antibodies, secondary antibodies, and labels can be the same or different from the first, second, or third porcine antibodies, porcine antigens, anti-porcine human antibodies, secondary antibodies, and labels.

Except as specifically indicated, discussions herein with respect to support 104 apply to supports 104', 104" or fourth etc. supports; discussion herein with respect to porcine antibody 102 apply to porcine antibodies 102', 102" or fourth etc. porcine antibodies; discussion herein with respect to porcine antigen 106 apply to porcine antigens 106', 106" or fourth porcine antigens; discussion herein with respect to anti-porcine human antigens 108 apply to anti-porcine human antigens 108', 108" or fourth anti-porcine human antigens; discussion herein with respect to secondary antibodies 110 apply to secondary antibodies 110', 110" or fourth etc. secondary antibodies; and discussion herein with respect to label 112 apply to label 112', 112" or fourth etc. labels.

The support 104 can be formed of any material that is appropriate for, or can be modified to be appropriate for, the attachment of porcine antibodies 102. For example, the support 104 can include a solid substrate or surface, comprising, for example, glass and modified or functionalized glass, plastics (including acrylics, polystyrene, methylstyrene, polyurethanes, Teflon^{™}, etc.), paramagnetic materials, thoria sol, carbon graphite, titanium oxide, latex or cross-linked dextrans such as sepharose, cellulose polysaccharides, nylon or nitrocellulose, ceramics, resins, silica or silica-based materials including silicon and modified silicon, carbon metals, inorganic glasses, optical fiber bundles, and a variety of other polymers.

In some implementations, the support 104 may be or include a well or an array of wells or depressions in a surface. These can be fabricated using techniques such as photolithography, stamping, molding, 3D printing, and micro-etching. In some implementations, a solid support can include a multiwell plate such as a 96-, 384- or 1536-well plate. In some implementations, the solid support can be located within a flow cell or flow cell apparatus (e.g., a flow cell on a Biacore^{™} chip or a protein chip).

The support 104 may also be or include a bead, sphere, particle, membrane, chip, slide, or test tube. Beads may include spheres or particles. Spheres, particles, or grammatical equivalents thereof may include discrete, non-planar particles, e.g., in the micrometer or nanometer dimensions. Spheres can include, for example, microspheres or nanospheres. Particles can include, for example, micro and nanoparticles. In some implementations a bead can be irregular in shape. Alternatively or additionally, the beads can be porous.

Example bead sizes range may from nanometers to millimeters, with beads from about 0.2 to about 200 microns being used in some implementations. In some implementations, beads can be micro beads where the bead size can range from about 0.5 to about 5 microns. In some implementations, beads smaller than 0.2 microns and larger than 200 microns can be used.

In some implementations, the support 104 may be or include a patterned surface (e.g., on a protein chip) suitable for immobilization of purified proteins (e.g., an antigen, an antibody) in an ordered pattern. An example patterned surface includes an arrangement of different regions in or on an exposed layer of a solid support. For example, one or more of the regions can be features where one or more purified proteins, such as antibody 102, are present. The features can be separated by interstitial regions where purified proteins are not present. In some implementations, the pattern can be an x-y format of features that are in rows and columns. In some implementations, the pattern can be a repeating arrangement of features and/or interstitial regions. In some implementations, the pattern can be a random arrangement of features and/or interstitial regions. Examples of patterned surfaces that can be used in the example assays and complexes are described in U.S. Patent Application Publication No. 2008/0280785, U.S. Patent Application Publication No. 2003/0153013, and PCT Publication No. WO2009/039170, which are incorporated herein by reference.

The supports 104 can comprise one or more isolated surfaces where for each surface a different anti-porcine antibody 102 is coupled or attached. For example, when beads are used as the support, each bead provides an isolated surface. In a well plate, each well can provide an isolates surface such as a bottom surface of a well. In patterned surface, the interstitial regions as described above can provide an isolated surface.

In some implementations, the porcine antibodies 102 may be coupled to the solid support 104 using one or more binding techniques such as covalent and non-covalent bonding. Covalent bonding techniques may include, but are not limited to, binding via carboxy groups, amide linkages, epoxy linkages, and binding through click chemistry. Covalent binding reagents can include any chemical or biological reagent that can be used to covalently immobilize an antigen directly on a surface of a solid support. Covalent binding reagents may include a carboxyl-to-amine reactive group such as carbodiimides, e.g., EDC (1-Ethyl-3-(3-Dimethylaminopropyl) Carbodiimide) or DCC (N,N'-dicyclohexylcarbodiimide), an amine reactive group such as N-hydroxysuccinimide (NHS) ester or imidoesters, a sulfhydryl-reactive crosslinker such as maleimides, haloacetyls or pyridyl disulfides, a carbonyl-reactive crosslinker group such as, hydrazides or alkoxyamines, a photoreactive crosslinker such as aryl azides or dizirines, or a chemoselective ligation group such as a Staudinger reaction pair. Click chemistry binding can include alkene and tetrazole photoclick reaction, alkene and azide [3+2] cycloaddition, alkene and tetrazine inverse-demand Diels-Alder, Copper(I)-catalyzed azide-alkyne cycloaddition (CuAAC), strain-promoted azide-alkyne cycloaddition (SPAAC), strain-promoted alkyne-nitrone cycloaddition (SPANC), and reactions of strained alkenes. Non-covalent bonding can employ any chemical or biological reagent that can be used to immobilize an antigen non-covalently on a surface of a solid support, such as affinity tags including biotin or capture reagents such as streptavidin or anti-tag antibodies, such as anti-His6 or anti-Myc antibodies.

Porcine antigens 106 are contacted with the anti-porcine antibodies 102 where antibody/antigen interactions form an antigen 106/antibody 102 complex tethered or attached to the support 104. The porcine antigens may be sourced from a single porcine donor. In some implementations, these are prepared from lysates of the porcine donor. Any blood, tissue, biological fluid, or organ can be used to prepare the lysates. For example, the lysates can be prepared from peripheral blood, spleen, or tissue of the porcine donor. Any useful method can be used to prepare the lysates. For example, for cell lysates, a suspension of cells can be collected by centrifugation and washed with a buffer such as PBS or with saline 2 to 3 times. For non-suspended cells, such as cells adherent to a container, trypsin treatment or scraping off adherent cells can be used to collect the cells. For tissues, the desired sample is dissected and then wash, such as with PBS buffer. After washing the tissue sample is homogenized, for example by sonication. For suspended cells and tissues, the final steps are similar: lysis buffer is added, the sample is sonicated, and then centrifuged to provide the lysate as the supernatant. In some implementation, a kit includes Instructions for preparing lysates from the porcine donor source and contacting the lysates with the anti-porcine antibodies 102. The instructions can be in text form, such as on a sheet, tag or on packaging of a kit. The instructions can also be on a website associated with a kit, such as accessible through a bar code or website address on the kit, a card/sheet in or of the kit, or on packaging for the kit.

After forming the antigen 106/antibody 102 complex, these are washed to remove any unbound proteins and other material in the porcine lysate. Washing can be in a constant flow or batch like, with 1, 2, 3 or more wash steps. Washing solutions can be a buffer such tris (hydroxymethyl)aminomethane (Tris)-based buffers like Tris-buffered saline (TBS) or phosphate buffers like phosphate-buffered saline (PBS). Wash buffers can be composed of detergents, such as ionic or non-ionic detergents. In some implementations, the wash buffer can be a PBS buffer at about pH 7.4 and may include a polysorbate-type nonionic surfactant, such as that provided by Sigma-Aldrich (St. Louis, MO) under the tradename Tween^{®}20, at about 0.05%.

To detect porcine antigens from a human, a biological sample from a human is contacted with the antigen 106/antibody 102 complex. The biological sample can, for example, be sourced from a biological fluid, such as blood, serum, plasma, lymph, urine, or from a tissue of the human subject. In some implementations, the biological sample is sera, plasma, lymph fluid, or biopsy aspirates. The human can be a subject that has received an organ from the porcine donor, or a subject who is being considered for receiving an organ from the porcine donor.

Unbound proteins and other material in the biological sample is removed by washing, such as with a constant flow or batch like using wash solutions as previously described. The secondary antibody 110 is then contracted with the "sandwich" structure of porcine antibody 102/ porcine antigen 108/ human antibody 108. The secondary antibody 110 includes a label which is used to detect the secondary antibody 110 and concomitantly human antibody 108.

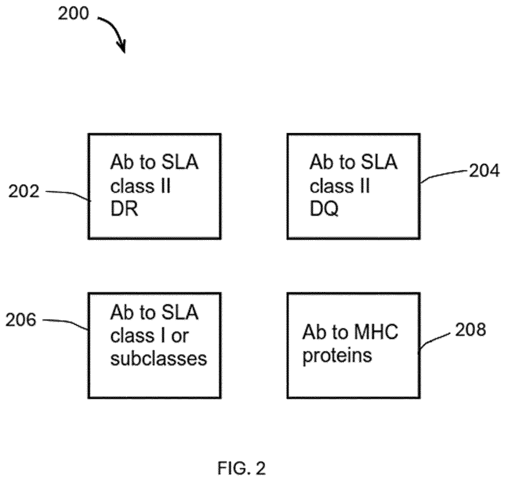

In some implementations, porcine antibodies 102 include one or more populations of anti-porcine antibodies, 200 as illustrated in FIG. 2. Examples of porcine antibodies 104 that can be attached to the solid support 104 include, but are not limited to, a first population 202 of anti-porcine antibodies directed against SLA Class II DR, a second population 204 of anti-porcine antibodies directed against SLA Class II DQ, and a third population 206 of anti-porcine antibodies directed against SLA Class I or an SLA subclass selected from SLA1, SLA2, SLA3, SLA6, SLA7, or SLA8. In some implementation, a fourth population 208 of anti-porcine antibodies comprising anti-MHC antibodies which are directed against major histocompatibility complexes (MHC) proteins are included. Additional populations of antibodies to porcine antigens/proteins can be used where human antibodies can react/attach to the porcine antigens/proteins.

Any antibodies directed to the classes or proteins described above can be used in the first, second, third and fourth populations may be used. For example, and not limited to these, the first population can include BL2H5 or BL4H2; the second population can include 2F4, 1F12 or 2E9/13; the third population can include JM1E3; and the fourth population can include 12E7, a monoclonal antibody that recognizes MIC2. These antibodies are well-known and readily available to the skilled person (see, e.g. Bullido R. et al., "Characterization of five monoclonal antibodies specific for swine class II major histocompatibility antigens and crossreactivity studies with leukocytes of domestic animals", Developmental & Comparative Immunology, vol 21(3), 1997; Galiani, D. et al., "A new monoclonal antibody (JM1E3) specific for porcine SLA Class I antigen recognises HLA Class I antigens and interferes with HLA recognition by human NK inhibitory receptors", In Leucocyte Typing VII. Edited by Mason. D. et al. Oxford University Press pp 437-39. 2002; Latron F. et al., "Immunochemical characterization of the human blood cell membrane glycoprotein recognized by the monoclonal antibody 12E7", Biochem J., vol. 247(3), pp. 757-64, 1987).

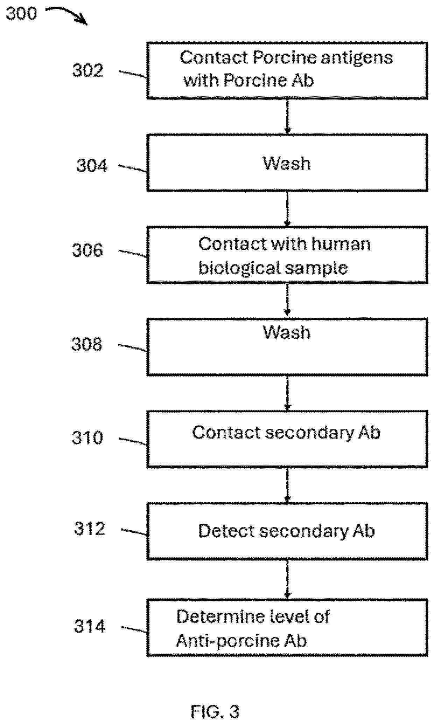

FIG. 3 is a flowchart showing steps included in an example process or process (assay) 300 for detecting anti-porcine antibodies. The steps include contacting 302 porcine antigens with porcine antibodies. This may be performed by contacting a solution such as a porcine lysate with a fluid containing the porcine antibodies 102 (FIG. 1) attached to the solid support 104. Unbound porcine antigens are removed 304 by washing as previously described. The antibody 102/antigen 106 complex and any bound porcine antigens 106 are contacted with a human biological sample 306. This may also be preformed by contacting the biological sample in a solution of the complexes attached to solid support 104. Unbound human antigens and proteins are removed 308 by washing as previously described. The composition is then contacted 310 with a secondary antibody 110. The secondary antibody can be directed to either human IgG or human IgM. The secondary antibody 110 includes the label 112. In some implementations the label 112 is a dye. In other implementations the label 112 is an enzyme such as HRP-streptavidin. The label 112 is detected in step 312 and the relative or absolute amount of anti-porcine antibodies 108 in the sample is determined 314 based on the level of the detected label 112.

In some implementations, at least three or populations of porcine antibodies as previously described are coupled each to an isolated surface of the solid support 104.

A signal generated by the label 112 can be detected using any suitable method known in the art. Exemplary methods can include, but are not limited to, visual detection, fluorescence detection (e.g., fluorescence microscopy), scintillation counting, surface plasmon resonance, ellipsometry, atomic force microscopy, surface acoustic wave device detection, autoradiography, and chemiluminescence. As one of skill in the art will appreciate, the choice of detection method will depend on the specific labeling agent employed. The relative or absolute amount of anti-porcine antibodies 108 is determined by comparing the signal generated by label 112 to a control or predefined cutoff.

In some implementations, the detecting is carried out by measuring a fluorescence intensity. Such methods are generally known in the art. For example, the xMAP Technology by Luminex (Luminex Corp., Austin, Tex.) allows one to perform up to 500 immunoassays in varying combinations in a single reaction in a standard 96-well microplate. In an example implementation, two or more solid supports such as beads (for example, two, three, four, five, six, and so forth), each coupled with anti-porcine antibodies, are mixed together and then filled in tubes which may be part of the cartridge or provided in a vial. For example, a first population 202 of anti-porcine antibodies directed against SLA Class II DR is coupled to a first solid support (e.g., Bead ID 001), a second population 204 of anti-porcine antibodies directed against SLA Class II DQ is coupled to a second solid support (e.g., Bead ID 002), and a third population 206 of anti-porcine antibodies directed against SLA Class I or an SLA subclass selected from SLA1, SLA2, SLA3, SLA6, SLA7, or SLA8, is coupled to a third solid support (e.g., Bead ID 003). Optionally, a fourth population 208 of anti-porcine antibodies comprising anti-MHC antibodies which are directed against major MHC are coupled to a fourth solid support (e.g. Bead ID 004). In this example, the different Bead IDs contain two different fluorescent dyes in differing ratios such that a Luminex instrument is able to distinguish among the different Bead IDs, and thereby can distinguish amount the different antibodies attached. In some embodiments, the detecting is carried out by an immunological analysis.

In some implementations, the detecting is carried out by the labeled secondary antibody 112. Labeled secondary anti-human antibodies are commonly used in the art and available from various vendors. In some embodiments, the secondary anti-human antibodies are labeled by enzyme conjugates. Non-limiting examples include alkaline phosphatase (AP) or horseradish peroxidase (HRP). In some embodiments, the secondary anti-human antibodies are labeled by fluorescent conjugates. Non-limiting examples include fluorescein (FITC), tetramethylrhodamine (TRITC), Alexa Fluor, phycoerythrin, etc. In some embodiments, the secondary anti-human antibodies are labeled by biotin.

Also described herein are example implementations of kits for use in determining the risk of transplant rejection or injury in a human subject receiving or being considered for receiving a transplantation from the porcine donor

The term "antibody" is used interchangeably with "immunoglobulin" (Ig). An example antibody includes a polypeptide product of B-cells or recombinant equivalents thereof, that is able to bind to a specific molecular antigen and is composed of two heavy chains and two light chains. Each amino-terminal portion of each chain includes a variable region that confers binding specificity. See Borrebaeck (ed.), Antibody Engineering, Second Edition, Oxford University Press (1995); and Kuby, Immunology, Third Edition, W.H. Freeman and Company, New York (1997). Examples of antibodies include anti-porcine antibodies used to couple porcine antigens, which anti-porcine antibody/porcine antigen complexes are used as detection probes in the techniques and kits disclosed herein. An antibody can exhibit specific binding affinity where it binds to a single molecular species or pan-specific where it binds selectively to more than one related molecular species. The related molecular species can be a molecular antigen that can be bound by the target antibody. An example antibody can be derived from any mammalian organism, including human, mouse, rabbit, goat, chicken, donkey, pig or the like. In some implementations, porcine antibodies coupled to porcine antigens are used in a detection probe to detect human antibodies. Furthermore, antibody can be monoclonal, polyclonal, chimeric or humanized. The antibodies identified herein can also be used in the assays and kits described herein.

The term "signal-to-noise ratio" includes a ratio between one protein's specific binding affinity to its target and the protein's non-specific binding affinity to a non-target. A non-target is a protein such as bovine serum albumin and human serum albumin. A negative control support such as a bead can be coated with the non-target to determine the non-specific binding affinity. In some implementations, binding between an antigen and an antibody, including binding of human antibodies to porcine antigens, exhibits a standard score for a signal-to-noise ratio of 25 or less.

Antibodies, such as those used as part of the detection probes, may include full length antibodies as well as functional fragments such as those antibodies. An example functional fragment, when used in reference to an antibody, incudes to a portion of the antibody including, e.g., heavy or light chain polypeptides that retain some or all of the binding activity as the antibody from which the fragment was derived. Such functional fragments can include, for example, an Fd, Fv, Fab, F(ab'), F(ab) 2, F(ab') 2, single chain Fv (scFv), diabody, triabody, tetrabody and minibody, which can be found described in, for example, Harlow and Lane, Antibodies: A Laboratory Manual, Cold Spring Harbor Laboratory, New York (1989); Myers (ed.), Molec. Biology and Biotechnology: A Comprehensive Desk Reference, New York: VCH Publisher, Inc.; Huston et al, Cell Biophysics, 22:189-224 (1993); Pluckthun and Skerra, Meth. Enzymol., 178:497-515 (1989) and in Day, E.D., Advanced Immunochemistry, Second Ed., Wiley-Liss, Inc., New York, NY (1990). Antibody functional fragments such as these can be used in the assays described herein.

Example antibody specific binding polypeptides include any polypeptide that can specifically recognized antibodies as well as functional fragments thereof. Examples of antibody specific binding polypeptides include IgG binding proteins, receptors, chimeric receptors and binding polypeptides identified from screening of random or combinatorial libraries.

Examples of IgG binding proteins include protein A and protein G. Antibody specific binding polypeptides may be chemically synthesized or purified from a natural source or recombinantly made. The antibody specific binding polypeptides described herein can be mammalian, including mouse, rabbit, goat, chicken, donkey, pig and the like. In some implementations, the antibody specific binding polypeptides are porcine antigens and proteins or fragments thereof.

An example label includes a molecular entity that emits a signal and can be used as a readout or measurement for detection of an analyte. Various classes of labels exist. Examples of these classes include a flurophore, an enzyme, a chemiluminscent moiety, a radioactive moiety, an organic dye, a small molecule, a polypeptide or functional fragment thereof. Examples of fluorophores include fluorescent dyes like phycoerytherin (PE), fluorescein isothiocyanate (FITC), tetramethylrhodamine (TRITC), BODIPY and AlexaFluor^{®} dyes. Fluorescent dyes can also include fluorescence resonance energy transfer (FRET)-dyes or time-resolved (TR)-FRET dyes. Fluorophore labels also include fluorescent proteins such as green fluorescent protein (GFP) and cyan fluorescent protein (CFP). Examples of enzyme labels include alkaline phosphatase (AP) or horseradish peroxidase (HRP). When any of the substrates S^'S^'-Tetramethylbenzidine (TMB), 3,3'-Diaminobenzidene (DAB) or 2,2'-azino-bis(3- ethylbenzothiazoline-6-sulphonic acid) (ABTS) are applied to HRP, a colored (chromogenic) or light (chemiluminescent) signal is produced. Radioactive labels include, for example carbon-14 or Tritium, for example. Small molecule labels include biotin, resins such as agarose beads and fluorescently labeled magnetic beads or nanoparticles such as colloidal gold. Polypeptide or functional fragment labels include Avidin, Streptavidin or NeutrAvidin which have an affinity for biotin. Polypeptide or functional fragment labels also include hemagglutinin (HA), glutathione-S-transferase (GST) or c-myc. The secondary antibodies and labels described herein can be used in the assays described herein to detect the antibodies bound to the antigens. An example kit can include a solid support, examples of which are described herein. The kit can also include a control. An example control includes a positive or negative standard for comparison against a tested sample where the existence or amount of anti-porcine human antibody is unknown. A kit can include a positive control. In some implementations, a positive control includes a sample containing a detectable amount of an anti-porcine human antibody or functional fragment thereof or levels above the threshold. In some implementations, a positive control can be obtained from a subject who has levels of anti-porcine human antibody above threshold. Additionally or alternatively, a positive control can contain antibody or functional fragment thereof specific to an antigen such as SLA. The antibody or functional fragment thereof can be selected from a monoclonal or polyclonal antibody. In other implementations, the kit can include a negative control. A negative control can include a sample containing no detectable amount of anti-porcine human antibody or functional fragment thereof or levels below the threshold. In some implementations, a negative control can be obtained from a control individual or can be synthesized in vitro. For example, a negative control can include water or buffer.

An example kit can include one or more ancillary reagents. An example ancillary reagent includes a substance, mixture, material or component that is useful to implement an assay or composition. Example ancillary reagents include a conjugation reagent, a buffer, instructions, instruments and the like. In some implementations, the kit can include instructions on how to prepare lysates such as porcine lysates for use with the kit.

In some implementations, a kit can include a reagent and the reagent used in the kit can include any conjugation reagent known in the art, including covalent and non-covalent conjugation reagents. Covalent conjugation reagents can include any chemical or biological reagent that can be used to covalently immobilize a polypeptide on a surface. Covalent conjugation reagents can include a carboxyl-to-amine reactive group such as carbodiimides such as EDC or DCC, an amine reactive group such as N-hydroxysuccinimide (NHS) ester or imidoesters, a sulfhydryl-reactive crosslinker such as maleimides, haloacetyls or pyridyl disulfides, a carbonyl- reactive crosslinker groups such as, hydrazides or alkoxyamines, a photoreactive crosslinker such as aryl azides or dizirines or a chemoselective ligation group such as a Staudinger reaction pair. Non-covalent immobilization reagents can include any chemical or biological reagent that can be used to immobilize a polypeptide of this disclosure non-covalently on a surface, such as affinity tags such as biotin or capture reagents such as streptavidin or anti-tag antibodies, such as anti-His6 or anti-Myc antibodies.

An example reagent can include combinations of conjugation reagents. Such combinations include, e.g., EDC and NHS, which can be used, e.g., to immobilize a protein on a surface, such as a carboxylated dextrane matrix (e.g., on a BIAcore^{™} CM5 chip or a dextrane-based bead). Combinations of conjugation reagents can be stored as premixed reagent combinations or with one or more conjugation reagents of the combination being stored separately from other conjugation reagents.

A reagent used in a kit can include a coating buffer. A coating buffer can include sodium carbonate-sodium hydroxide or phosphate. In some implementations, the coating buffer can be 0.1M NaHCCb (e.g, about pH 9.6).

In some implementations, the reagent can include a wash buffer as previously described. In some implementations, the reagent can include a dilution buffer. Any dilution buffer known in the art can be included in the kit. Examples of dilution buffers include a carrier protein such as bovine serum albumin (BSA) and a detergent such as Tween^{®}20. In some implementations, the dilution buffer can be PBS at about pH 7.4 including BSA at about 1% BSA and Tween ^{®}20 at about 0.05%.

In some implementations, the reagent can include a detection or assay buffer. Any detection or assay buffer known in the art can be included in the kit.

The detection or assay buffer can be a colorimetric detection or assay buffer, a fluorescent detection or assay buffer or a chemiluminescent detection or assay buffer. Colorimetric detection or assay buffers include, but are not limited to, PNPP (p-nitrophenyl phosphate), ABTS (2,2'-azino-bis(3- ethylbenzothiazoline-6-sulphonic acid)), or OPD (o-phenylenediamine). Fluorescent detection or assay buffers include phycoerythrin, Cy3, Alexa Fluur 555, QuantaBlu^{™} or QuantaRed^{™} (Thermo Scientific, Waltham, MA). Chemiluminescent detection or assay buffers can include luminol or luciferin. Detection or assay buffers can also include a trigger such as H₂O₂ and a tracer such as isoluminol-conjugate.

In some implementations, the reagent can include solutions useful for calibration or testing. In some implementations, the reagent can include a stop solution. Any stop solution known in the art can be included in the kit. Example stop solutions terminate or delay the further development of the detection reagent and corresponding assay signals.

Stop solutions can include, e.g., low-pH buffers (e.g., glycine-buffer, pH 2.0), chaotrophic agents (e.g,. guanidinium chloride, sodium-dodecyl sulfate (SDS)), or reducing agents (e.g., dithiothreitol, b-mecaptoethanol) or the like.

In some implementations, the reagent can include cleaning reagent(s). Cleaning reagents can include any cleaning reagent known in the art. In some implementations, the cleaning reagents can be the cleaning reagents recommended by the manufacturers of the automated assay systems.

In some implementations, a kit of the type described herein can be used to perform an assay on a test instrument such as the test instruments or systems described herein. In some implementations, detection instruments are configured to detect and to measure a label of a secondary antibody. To this end, the detection instruments are configured for detecting or measuring fluorescence, luminescence, chemiluminescence or absorbance, reflectance, transmittance, birefringence or refractive index. In some implementations, detection instruments can implement confocal and non-confocal microscopy, a microplate reader, a flow cytometer and the like.

In some implementations, the kit can include a component suitable for collecting a biological sample as described below. A component suitable for collecting a biological sample can include collection tubes, columns, syringes, needles and the like. In some implementations, the kit can include instructions for using the components of the kit. Instructions can be in any form, inside or outside of the kit. The instructions provide details regarding protocol and analytical techniques.

Components of a kit can be in varying physical states. For example, some or all of the components can be lyophilized or in aqueous solution or frozen. Such components may include one or more antigens or antibodies, attached to a solid support as described herein, the solid support, a detection probe, and ancillary reagents.

A kit of the type described herein can be tailored to specific assay technologies. For example, in some implementations, the kits can be a FIA kit, a CIA kit, a RIA kit, a multiplex immunoassay kit, a protein/peptide array immunoassay kit, a SPRIA kit, an IIF kit, an ELISA, a PMAT kit or a Dot Blot kit. In some implementations, the ELISA kits can include a washing buffer, a sample diluent, a secondary antibody-enzyme conjugate, a detection reagent and a stop solution. In some implementations, the Dot Blot kits can include a washing buffer, a sample diluent, a secondary antibody-enzyme conjugate, a detection reagent and a stop solution. In some implementations, the CIA kit can include a washing buffer, a sample diluent, a tracer (e.g., isoluminol-conjugate) and a trigger (e.g., H2O2). In some implementations, the multiplex kit can include a washing buffer, a sample diluent and a secondary antibody-enzyme conjugate. In some implementations, the kits can be tailored to the Luminex platform and include, as an example, xMAP^{®} beads.

In some implementations, a porcine antigen can be bound by anti-porcine human antibody with high affinity; or an anti-porcine antibody can be bound by porcine antigen with high affinity. Higher binding affinities are evidenced by lower dissociation constants (KDS) for the antigen X:anti-X antibody complex or by higher association constants (KAS) for the respective anti-X antibody and antigen X. In some implementations, the dissociation constants for (KDS) for the porcine antigen:anti-porcine antibody complex can be less than 1 mM, less than 300 nM, less than 100 nM, less than 30 nM, less than 10 nM, less than 3 nM, less than 1 nM, less than 300 pM, less than 100 pM, less than 30 pM, less than 10 pM, less than 3 pM or less than 1 pM. Techniques for measuring binding affinities of antibodies to antigens are known and include ELISA, isothermal titration calorimetry (ITC), microfluidic diffusional sizing, flow-induced dispersion analysis and surface plasmon resonance (SPR).

Also described herein are techniques for determining the risk of transplant rejection in a human subject receiving or being considered for receiving a transplantation from the porcine donor. Such techniques include: (a) contacting a biological sample from a human subject who will or has undergone a xenotransplant, including a subject seronegative for known xenotransplant-related antibodies, with an porcine antigen described herein, and (b) detecting the presence of the anti-porcine human antibody in the biological sample. A presence of the bound anti-porcine human antibody is or may be indicative of the risk of transplant rejection.

In some implementations, detection of an increased level of anti-porcine human antibody compared to a control individual indicates that the subject is at risk of developing clinical symptoms of a transplant rejection. In some implementations, a subject can be at risk of developing clinical symptoms of transplant rejection associated with the level of anti-porcine antibodies within less than 3 months, less than 6 months, less than 9 months, less than 12 months, less than 18 months, less than 2 years, less than 3 years, less than 4 years, less than 5 years, less than 6 years, less than 7 years, less than 8 years, less than 9 years, less than 10 years, less than 12 years, less than 14 years, or less than 16 years from the determination of the increased anti-porcine human antibody level.

In some implementations, the presence of an increased level of anti-porcine human antibody compared to a healthy control individual indicates that the subject is more than 5%, more than 10%, more than 15%, more than 20%, more than 25%, more than 30%, more than 35%, more than 40%, more than 45%, more than 50%, more than 60%, more than 70% or more than 80%, or more than 90% likely to develop clinical symptoms of a transplant rejection associated with anti-porcine human antibody within 5 years following the determination of increased anti-porcine human antibody. In some implementations, the presence of an increased level of anti-porcine human antibody can indicate that the subject is more than 2-fold, more than 3-fold, more than 4-fold, more than 5-fold, more than 6-fold, more than 7-fold, more than 8- fold, more than 9-fold, or more than 10-fold likely to develop clinical symptoms of a transplant rejection associated with anti-porcine human antibody within 5 years following determination of increased anti-porcine human antibody level compared to a healthy control individual.

The terms "subject," "organism," "individual" or "patient" are used as synonyms and interchangeably and may refer to a vertebrate mammal. As an example, the subjects of this disclosure can include healthy human subjects, asymptomatic human subjects and human diseased subjects.

A subject can be "suspected of being at risk of transplant rejection associated with anti-porcine human antibody" as determined by the presence of certain risk factors that are known in the art. Example risk factors include, for example, a genetic predisposition, a personal disease history, a lifestyle factor, an environmental factor, a diagnostic indicator and the like.

A subject at risk of transplant rejection or injury associated with anti-porcine human antibody can have a genetic predisposition for developing a transplant rejection associated with anti-porcine human antibody. The subject can be exposed to certain lifestyle factors (e.g., smoking cigarettes) or environmental factors promoting the development of transplant rejection associated with anti-porcine human antibody. The subject can show clinical manifestations of transplant rejection associated with anti-porcine human antibody. The subject can be a patient who is receiving a clinical workup to diagnose transplant rejection associated with anti-porcine human antibody or to assess the risk of transplant rejection associated with anti-porcine human antibody.

In some implementations, the subject can have anti-porcine human antibodies in their bodily fluid (e.g., blood) or tissue. In some implementations, the subject can have elevated anti-porcine human antibodies in their bodily fluid or tissue compared to normal healthy subjects. In some implementations, the subject will not have elevated anti-porcine human antibodies in their bodily fluid or tissue compared to normal healthy subjects.

In some implementations, the subject can be treatment naive. In some implementations, the subject can be undergoing treatments to avoid transplant rejection associated with anti-porcine antibodies (e.g., drug treatments). In some implementations, the subject can be in remission. In some implementations, the remission can be drug-induced. In some implementations, the remission can be drug-free.

A subject can be "seronegative for known anti-porcine human antibodies associated with a transplant rejection" as determined by the failure to detect the presence of known anti-porcine human antibodies associated with a transplant rejection in a blood test.

In some implementations, the known anti-porcine human antibodies associated with a disease can be directed against SLAs or an MHC like protein.

In some implementations, the subject can be asymptomatic. Asymptomatic subjects include healthy subjects who have essentially no risk or only a low risk of transplant rejection associated with an anti-porcine human antibody (e.g., there is a less than 10%, less than 5%, less than 3% or less than 1% probability that the asymptomatic patient will have a transplant rejection associated with an anti-porcine human antibody over the following five year period). Asymptomatic subjects further include healthy subjects who have a high risk of a transplant rejection associated with anti-porcine antibodies. (e.g., there is a greater than 50%, greater than 70%, greater than 90%, or greater than 95% probability that the asymptomatic patient will have a transplant rejection associated with anti-porcine antibodies over the following five year period). Asymptomatic subjects further include subjects, who can display mild early diagnostic indicators of a transplant rejection associated with anti-porcine antibodies, but who are otherwise risk or complaint free.

It should be noted that the terms "healthy control individual," "healthy subjects," and grammatical equivalents thereof are used interchangeably and refer to subjects who do not have increased anti-porcine antibody levels above baseline or a standard known or determined to represent subjects who do not have a risk of transplant rejection associated with anti-porcine antibodies.

In some implementations, the healthy subjects can have never suffered from a transplant rejection. In some implementations, the healthy subjects can have had a previous organ transplant. In some implementations, the healthy subjects can be undergoing a routine medical checkup. In some implementations, the healthy subjects can be members of a control group in, for example, a clinical trial. In some implementations, the healthy subjects can be at risk of a transplant rejection, as determined by the presence of certain risk factors that are well known in the art. Such risk factors include, without limitation, a genetic predisposition, a personal disease history, a familial disease history, a lifestyle factor, an environmental factor, a diagnostic indicator and the like.

The baseline or standard that determines or defines a subject as a subject not suffering from a transplant rejection associated with anti-porcine antibodies is the reference interval. In diagnostic or health-related fields, the reference interval is a range of values observed in the reference subjects, which can be healthy control individuals, designated by specific percentiles. The reference interval can be any range of values as determined by those having skill in the art. See CLSI, "How to define and determine reference intervals in the clinical laboratory: approved guideline," C28:A2 (2000).

In some cases, the reference interval can be stringent or less stringent depending on the specific analyte being measured or type of organ transplant. in some implementations, the reference interval can be set at the 95th percentile. In order to increase specificity and sensitivity, e.g., increase stringency, a higher cut-off can be used such as the 96th percentile or the 97th or the 98th or the 99th.

An anti-porcine human antibody can be considered increased in a subject if anti-porcine human antibody levels are at least above the 95th percentile relative to anti-porcine human antibody levels in healthy control subjects. In other implementations, anti-porcine human antibody can be considered increased in a subject if anti-porcine human antibody levels are above the 96th, 97th, 98^{th}, or 99th percentile. A subject with anti-porcine human antibody levels at or above any of the disclosed reference intervals is considered to be at a higher risk have a transplant rejection associate with anti-porcine human antibody.

In some implementations, the presence of anti-porcine human antibody can be based on a comparison of signal against background in a healthy subject. In some implementations, the presence of anti-porcine human antibody can be increased or decreased relative to an average or median anti-porcine human antibody level observed in a population of healthy subjects. In some implementations, anti-porcine human antibody can be absent in healthy subjects. In some implementations, anti-porcine human antibody level cannot be detected above the noise of the respective assay used to determine anti-porcine human antibody level. In some implementations anti-porcine human antibody can be considered present in a sample if an anti-porcine human antibody level can be detected above the noise of the respective assay used to determine an anti-porcine human antibody level. In some implementations, anti-porcine human antibody can be considered increased in a sample if the signal in an anti-porcine human antibody detection assay is at least two standard deviations above noise such as the average or mean signal for control samples. In some implementations, anti-porcine human antibody can be considered present in a sample if the level of anti-porcine human antibody exceeds a predetermined threshold level. An anti-porcine human antibody threshold level can be determined by a skilled artisan, such as a clinical physician, based on a variety of factors, such as the specific objectives of a clinical trial or the diagnostic and prognostic significance of a certain anti-porcine human antibody level or the results of another diagnostic test for a disease associated with an anti-porcine human antibody that does not involve the detection of anti-porcine human antibody levels.

Anti-porcine human antibody can be detected in a variety of different biological samples obtained from a subject. Examples of "biological sample" include any specimen from the body of an organism that can be used for analysis or diagnosis. As an example, a biological sample can include a liquid sample such as whole blood, plasma, serum, synovial fluid, amniotic fluid, sputum, pleural fluid, peritoneal fluid, central spinal fluid, urine, bile, saliva or tears. A biological sample can also include a solid tissue sample such as a liver biopsy, bone marrow, buccal or other solid or semi-solid aggregated of cells. In the context of the present disclosure, a biological sample obtained from a subject can be any sample that contains or is suspected to contain autoantibodies and encompasses any material from a subject in which an anti-porcine human antibody can be detected.

A biological sample can be or include, for example whole blood, serum, plasma or sputum. In some implementations, a biological sample of the present disclosure can be a tissue biopsy such as a liver, heart, kidney, lung biopsy. The biopsy can be less than 10 mm, less than 11 mm, less than 12 mm, less than 13 mm, less than 14 mm, less than 15 mm, less than 16 mm, less than 17 mm, less than 18 mm, less than 19 mm, less than 20 mm, less than 21 mm, less than 22 mm, less than 21 mm, less than 23 mm, less than 24 mm or less than 25 mm in length. The liver biopsy can include, in addition to the lengths disclosed herein, at least 4 portal triads, at least 5 portal triads, at least 6 portal triads, at least 7 portal triads, at least 8 portal triads, at least 9 portal triads, at least 10 portal triads or at least 11 portal triads.

A biological sample can be obtained from any subject that contains or is suspected to contain anti-porcine human antibodies. A biological sample can also be obtained from any subject that does not or is not suspected to have anti-porcine human antibodies. Thus, in some implementations, a biological sample can be obtained from a symptomatic subject, an asymptomatic subject and a subject that is negative for anti-porcine human antibodies. In some implementations, the biological sample can be obtained from a mammal.

A biological sample can be collected and processed immediately or it can be collected, frozen and processed at a later date. The biological sample could also include a plurality of samples from one of the subjects described herein. In some implementations, the plurality of biological samples can be collected over periodically over the course of more than 12 hours, more than 1 day, more than 2 days, more than 3 days, more than 4 days, more than 5 days, more than 6 days, more than 7 days, more than 10 days, more than 14 days, more than 3 weeks, more than 1 month, more than 2 months, more than 3 months, more than 4 months, more than 5 months, more than 6 months, more than 9 months, more than 12 months, more than 18 months, more than 24 months, more than 30 months, more than 3 years months, more than 4 years, or more than 5 years.

In some implementations, the example techniques described herein can be performed by contacting the biological sample with 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, or 16 or more of the anti-porcine/porcine antigen complexes described herein.

In some implementations, anti-porcine human antibodies can be detected by immunoassay. Techniques and protocols for conducting immunoassays and biophysical protein-interaction assays are well known in the art. See, e.g., Wild D., The Immunoassay Handbook, Elsevier Science, 4th Edition (2013); Fu EL, Protein-Protein Interactions, Humana Press, 4th Edition (2004). Examples of immunoassays include fluorescent immunosorbent assay (FIA), a chemiluminescent immunoassay (CIA), a radioimmunoassay (RIA), multiplex immunoassay, a protein/peptide array immunoassay, a solid phase radioimmunoassay (SPRIA), an indirect immunofluorescence assay (IIF), an enzyme linked immunosorbent assay (ELISA) and a particle based multianalyte test (PMAT) or a Dot Blot assay.

In some implementations, the ELISA can be a sandwich ELISA. In some implementations, the sandwich ELISA can include the initial step of immobilizing a porcine antibody of this disclosure on a solid support as disclosed herein. For example, a porcine antibody can be immobilized on a wall of a microtiter plate well or of a cuvette.

An example "particle based multianalyte test (PMAT)" is includes an immunoassay that allows simultaneous measurement of two or more analytes in a single assay. For example, in PMAT, different types of particles are used simultaneously, with each type having immobilized a specific binding partner for a specific molecule species on the surface of its particles. In a solution, the analyte molecules to be detected are bound to their binding partners on the corresponding particle type. The binds are then detected optically through the addition of a secondary marker that marks all particle-bound analyte molecules of the multiplex assay. A PMAT can be performed using a variety of formats, such as flow cytometry, a capture sandwich immunoassay or a competitive immunoassay. For example, using a dual-laser flow-based detection instrument, the binding of analyte fractions, such as autoantibodies, can be detected through the fluorescence of the secondary marker. In another example, median fluorescence intensity (MFI) on particles is captured using a digital imager and analyzed using an algorithm to extract meaningful information for each analyte. In some implementations, the PMAT particle can be a bead. In effecting the PMAT, the presence of one or more autoantibodies specifically associated with an autoimmune disease can be identified and the patient can be diagnosed with the autoimmune disease that is specifically associated with the autoantibody identified by the PMAT.

In some implementations, a Dot-Blot or line immunoassay (LIA) can be used to detect anti-porcine human antibodies in a biological sample. Techniques and protocols for dot blot are well known in the art, including estimating polypeptide concentration. See Joint ProteomicS Laboratory (JPSL) of the Ludwig Institute for Cancer Research, Estimating protein concentration by dot blotting of multiple samples, Cold Spring Harbor Protocols, New York (2006).

The immunoassay can further include blocking steps, washing steps and additionally or alternatively, elution steps. Blocking steps can include contacting a solid support of the immunoassay in a blocking buffer for a sufficient time and temperature to allow blocking. Examples of blocking buffers are identified above. Washing steps include contacting a solid support of the immunoassay with a wash buffer to remove non-specific binding of polypeptides to the solid support. Exemplary washing buffers are described above.

Elution buffers can include any of a variety of elution buffers known in the art or disclosed herein. Elution buffers include, for example, a 0.1 M glycine:HCl solution between pH 2.5 and 3. Polypeptide complexes can be eluted from the solid support of the immunoassay to aid in detection and measurement of, for example, antigen X:anti-X antibody complexes.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

As used herein, "protein" or "polypeptide" or "peptide" include any peptide-linked chain of amino acids, which may or may not comprise any type of modification (e.g., chemical or post- translational modifications such as acetylation, phosphorylation, glycosylation, sulfatation, sumoylation, prenylation, ubiquitination, etc.).

As used herein, "subject" includes a mammal, including but not limited to a human, mouse, rat, or rabbit. The subject may be a human patient, such as a patient having a xenotransplant organ or a potential recipient of a xenotransplant organ.

As used herein, "sample" or "biological sample" include any sample comprising or being tested for the presence of anti-porcine human antibodies. Samples can include but are not limited to cellular extracts, extracellular fluid, fluid harvested from the body of a subject, culture media, blood, bone marrow, plasma, serum, biopsy, or any organ.

All publications cited herein are incorporated herein by reference in their entirety.

All examples described herein are non-limiting.

In the description and claims provided herein, the adjectives "first", "second", "third", and the like do not designate priority or order unless context suggests otherwise. Instead, these adjectives may be used solely to differentiate the nouns that they modify unless context suggests otherwise.

Elements of different implementations described may be combined to form other implementations not specifically set forth previously. Elements may be left out of the systems described previously without adversely affecting their operation or the operation of the system in general. Furthermore, various separate elements may be combined into one or more individual elements to perform the functions described in this specification. Other implementations not specifically described in this specification are also within the scope of the following claims.

### CLAUSES

For reasons of completeness, various aspects of the invention are set out in the following numbered clauses:
1. A kit for use in detecting porcine antigens in a biological sample from a human, the kit comprising:
   a. a support;
   b. anti-porcine antibodies coupled to the support;
   c. instructions for preparing lysates from a porcine donor source and contacting the lysates with the anti-porcine antibodies.
2. The kit of clause 1, wherein the porcine donor source is from peripheral blood, spleen or tissue of the porcine donor.
3. The kit of any one of the foregoing clauses, wherein the kit is for use in determining the risk of transplant rejection in a human subject receiving or being considered for receiving a transplantation from the porcine donor.
4. The kit of any one of the foregoing clauses, wherein the support comprises one or more isolated surfaces each surface containing a population of the anti-porcine antibodies, each population of anti-porcine antibody distinct from each other.
5. The kit of any one of the foregoing clauses, wherein a first population of anti-porcine antibodies is directed against SLA class II DR, a second population of porcine antibodies is directed against SLA Class II DQ, and a third population of porcine antibodies is directed against SLA Class I or an SLA subclass selected from SLA1, SLA2, SLA3, SLA6, SLA7, or SLA8.
6. The kit of any one of the foregoing clauses, wherein a fourth population of anti-porcine antibodies is directed against a non-SLA.
7. The kit of any one of the foregoing clauses, wherein the first population comprises an antibody selected from BL2H5 or BL4H2.
8. The kit of any one of the foregoing clauses, wherein the second population comprises an antibody selected from 2F4, 1F12 or 2E9/13.
9. The kit of any one of the foregoing clauses, wherein the third population comprises an antibody selected from JM1E3.
10. The kit of any one of the foregoing clauses, wherein the non-SLA is the antigen MIC2 and the fourth population is the antibody 12E7.
11. The kit of any one of the foregoing clauses, wherein the support comprises a well, a bead, or a patterned surface.
12. The kit of any one of the foregoing clauses, further comprising an anti-human antibody having a label attached thereto.
13. The kit of any one of the foregoing clauses, further comprising a lysate butter and wherein the anti-porcine antibodies are configured to bind to a porcine antigen present in a lysate from a porcine donor source.
14. A composition comprising:
   a. a first population of anti-porcine antibodies coupled to a first surface;
   b. a second population of anti-porcine antibodies coupled to a second surface; and
   c. a third population of anti-porcine antibodies coupled to a third surface.
15. The composition of clause 14, further comprising a fourth population of anti-porcine antibodies coupled to a fourth surface.
16. The composition of any one of clauses 14 to 15, wherein the first population of anti-porcine antibodies is directed against SLA class II DR, the second population or anti-porcine antibodies is directed against SLA Class II DQ, and the third population of anti-porcine antibodies is directed against SLA Class I or an SLA subclass selected from SLA1, SLA2, SLA3, SLA6, SLA7, or SLA8, the fourth population of porcine antibodies is directed against MHC proteins.
17. The composition of any one of clauses 14 to 16 further comprising first porcine antigens from a single porcine donor bound to the first population of anti-porcine antibodies, second porcine antigens from the single porcine donor bound to the second population of anti-porcine antibodies, and third porcine antigens from the single porcine donor bound to the third population of anti-porcine antibodies.
18. A method of detecting one or more anti-porcine antibodies in a biological sample from a human:
   a. contacting porcine lysate containing porcine antigens with the composition of any of clauses 14 to 17;
   b. removing any unbound porcine antigen;
   c. contacting a human biological sample containing human antibodies with the composition of step (b);
   d. removing any unbound human antibodies;
   e. contacting the composition of step (d) with a secondary antibody, the secondary antibody including a label;
   f. detecting the label; and
   g. determining the relative or absolute amount of anti-porcine antibodies in the sample based on the level of the detected label.
19. The method of clause 18 wherein the label is an enzyme or a dye, and wherein the biological sample is sera, plasma, lymph fluid, or biopsy aspirates.
20. The method of any of clauses 18 to 19, wherein the porcine lysate is sourced from peripheral blood, spleen or tissue of a single porcine donor.

## Claims

1. A kit for use in detecting porcine antigens in a biological sample from a human, the kit comprising:
a. a support;
b. anti-porcine antibodies coupled to the support;
c. instructions for preparing lysates from a porcine donor source and contacting the lysates with the anti-porcine antibodies.

2. The kit of claim 1, wherein the porcine donor source is from peripheral blood, spleen or tissue of the porcine donor.

3. The kit of any one of claims 1-2, wherein the support comprises one or more isolated surfaces each surface containing a population of the anti-porcine antibodies, each population of anti-porcine antibody distinct from each other.

4. The kit of any one of claims 1-3, wherein a first population of anti-porcine antibodies is directed against SLA class II DR, a second population of porcine antibodies is directed against SLA Class II DQ, and a third population of porcine antibodies is directed against SLA Class I or an SLA subclass selected from SLA1, SLA2, SLA3, SLA6, SLA7, or SLA8, and, optionally, a fourth population of anti-porcine antibodies is directed against a non-SLA.

5. The kit of claim 4, wherein:
- the first population comprises an antibody selected from BL2H5 or BL4H2, or
- the second population comprises an antibody selected from 2F4, 1F12 or 2E9/13, or
- the third population comprises an antibody selected from JM1E3.

6. The kit of claim 4, wherein the non-SLA is the antigen MIC2 and the fourth population is the antibody 12E7.

7. The kit of any one of claims 1-6, wherein the support comprises a well, a bead, or a patterned surface.

8. The kit of any one of claims 1-7, further comprising:
- an anti-human antibody having a label attached thereto, or
- a lysate buffer and wherein the anti-porcine antibodies are configured to bind to a porcine antigen present in a lysate from a porcine donor source.

9. Use of a kit as defined in any one of claims 1-8 for detecting porcine antigens in a biological sample from a human.

10. The use of the kit of claim 9, further comprising determining the risk of transplant rejection in a human subject receiving or being considered for receiving a transplantation from the porcine donor.

11. A composition comprising:
a. a first population of anti-porcine antibodies coupled to a first surface;
b. a second population of anti-porcine antibodies coupled to a second surface;
c. a third population of anti-porcine antibodies coupled to a third surface; and
d. optionally, a fourth population of anti-porcine antibodies coupled to a fourth surface.

12. The composition of claim 11, wherein the first population of anti-porcine antibodies is directed against SLA class II DR, the second population or anti-porcine antibodies is directed against SLA Class II DQ, the third population of anti-porcine antibodies is directed against SLA Class I or an SLA subclass selected from SLA1, SLA2, SLA3, SLA6, SLA7, or SLA8, and the fourth population of porcine antibodies is directed against MHC proteins.

13. The composition of any one of claims 11-12, further comprising first porcine antigens from a single porcine donor bound to the first population of anti-porcine antibodies, second porcine antigens from the single porcine donor bound to the second population of anti-porcine antibodies, and third porcine antigens from the single porcine donor bound to the third population of anti-porcine antibodies.

14. A method of detecting one or more anti-porcine antibodies in a biological sample obtained from a human, the method comprising:
a. contacting porcine lysate containing porcine antigens with the composition of any of claims 11 to 13;
b. removing any unbound porcine antigen;
c. contacting a human biological sample containing human antibodies with the composition of step (b);
d. removing any unbound human antibodies;
e. contacting the composition of step (d) with a secondary antibody, the secondary antibody including a label;
f. detecting the label; and
g. determining the relative or absolute amount of anti-porcine antibodies in the sample based on the level of the detected label.

15. The method of claim 14, wherein the biological sample is sera, plasma, lymph fluid, or biopsy aspirates, and optionally, wherein the label is an enzyme or a dye, and optionally, wherein the porcine lysate is sourced from peripheral blood, spleen or tissue of a single porcine donor.
